# EUROPÄISCHE PATENTSCHRIFT

(11) **EP 0 627 490 B1**
(45) Veröffentlichungstag und Bekanntmachung des Hinweises auf die Patenterteilung: **26.03.1997**
(21) Anmeldenummer: 94106283.8
(22) Anmeldetag: 22.04.1994
(51) Int. Cl.: C12P 19/14, C08B 37/18, A23L 1/09, A61K 31/715, C12S 3/02

(54) **Verfahren zur Herstellung von langkettigem Inulin**
Method for the preparation of long-chain inulin
Procédé pour la préparation d'inuline à longue chaîne

(30) Priorität: 17.05.1993 DE 4316425
(43) Veröffentlichungstag der Anmeldung: 07.12.1994
(73) Patentinhaber: SÜDZUCKER AKTIENGESELLSCHAFT MANNHEIM/OCHSENFURT, D-68165 Mannheim (DE)
(72) Erfinder: Kunz, Markwart, D-67550 Worms (DE); Munir, Mohammed, Dr., D-67271 Kindenheim (DE); Vogel, Manfred, Dr., D-67271 Neuleiningen (DE)
(74) Vertreter: Gleiss, Alf-Olav, Dr.jur. Dipl.-Ing.

(56) Entgegenhaltungen:
- EP-A- 0 201 676
- EP-A- 0 429 077
- EP-A- 0 440 074
- CARBOHYDRATE POLYMERS Bd. 9, Nr. 4 , 1988 , BARKING GB Seiten 277 - 285 J.F.KENNEDY ET AL. 'Analysis of the Oligosaccharides from the Roots of Arnica montana L., Artemisia absinthium L., and Artemisia dracuncula L.'

## Beschreibung

Die Erfindung betrifft die Herstellung von langkettigem Inulin aus pflanzlichen Extrakten bei gleichzeitiger Gewinnung von Glucose und Fructose.

Inulin ist ein zur Gruppe der Fructane gehörendes Polysaccharid. Es kommt in wirtschaftlich gewinnbaren Mengen in verschiedenen Pflanzen wie Topinambur- und Dahlienknollen sowie in Zichorienwurzeln vor. Es ist ein Heterofructan, da es an einer β-2-1- verknüpften Kette von Fructosemolekülen als Abschluß eine α-D-Glucose am reduzierenden Ende trägt. Die Kettenlänge hängt sowohl von der Pflanzenart als auch von der Wachstumsphase der Pflanze ab.

Es ist bekannt (F. Perschak und L. Wolfslehner, Zuckerind., 115 (1990) 466-470), Inulin mit heißem Wasser aus dem Pflanzengewebe zu extrahieren, den Extrakt zu entsalzen und zu entfärben und daraus Inulin in trockener Form zu gewinnen. Dieses Inulin, das neben dem polymeren Anteil noch Monosaccharide wie Glucose und Fructose, Disaccharide wie Saccharose und Fructooligosaccharide enthält, dient als Rohstoff für die erfindungsgemäße Herstellung von langkettigem Inulin. Es wird im folgenden als "Rohinulin" bezeichnet.

Aus der DE-A 40 03 140 ist dagegen bekannt, Inuline enzymatisch in kürzere Ketten zu spalten und die Fraktion mit einem Polymerisationsgrad von ca. 3-7 als kalorienreduzierte und diabetikergeignete Zuckeraustauschstoffe zu verwenden.

Aufgrund seines Mono- und Disaccharidgehaltes ist das Rohinulin für die Herstellung von diätetischen Lebensmitteln, insbesondere solche die für Diabetiker vorgesehen sind, ungeeignet. Auch kurzkettige Oligosaccharide können aufgrund ihrer Hygroskopizität bzw. Klebrigkeit bei der Verwendung von Rohinulin in Lebensmitteln sowohl bei der Verarbeitung als auch bei der Lagerung sehr stören.

Für eine Reihe von Anwendungen im Lebensmittelbereich (z.B. Fleischwaren) stört der Süßgeschmack der im Rohinulin enthaltenen kurzkettigen Oligosaccharide. Da langkettiges Inulin geschmacksneutral ist, ist es für solche Anwendungen besser geeignet als das Rohinulin.

Auch bei der chemischen bzw. biochemischen Umsetzung von Rohinulin werden, bedingt durch den Gehalt an Mono-, Di- und Oligosacchariden, nur undefinierte Produktgemische erhalten, die sich praktisch nicht aufreinigen lassen.

Deshalb ist es wünschenswert, aus dem Rohinulin durch Abtrennung der kurzkettigen Oligosaccharide ein Inulinprodukt mit nur langkettigen Molekülen zu erhalten. Dabei werden hier als kurzkettige Oligosaccharide auch noch Moleküle verstanden, die lymerisationsgrade (DP) von bis zu 10 - 12 aufweisen. Langkettiges Inulin ist dementsprechend nahezu frei von Molekülen mit DP < 10 - 12 und weist somit im Falle von Zichorieninulin eine mittlere Kettenlänge von > 20 auf.

Zur Herstellung eines solchen langkettigen Inulins kommen verschiedene Verfahren in Frage.

Langkettiges Inulin läßt sich aus wässriger Lösung in Gegenwart hoher Konzentrationen an organischen Lösungsmitteln wie Methanol, Ethanol, Isopropanol oder deren Gemischen unter geeigneten Bedingungen ausfällen und dann mit einer Zentrifuge oder Drucknutsche isolieren. Dieses Verfahren hat einerseits den Nachteil, daß man mit organischen Lösungsmitteln arbeiten muß, deren Einsatz besonders im Lebensmittelbereich äußert problematisch ist. Andererseits muß man mit großen Volumina arbeiten, wodurch die abgetrennten gelösten Anteile wie Glucose, Fructose, Saccharose und Oligosaccharide aus verdünnten Lösungen zurückzugewinnen sind und die Ausbeute an den gewünschten langkettigen Inulinen durch Lösungsverluste gering ist.

Wässrige Inulinlösungen können auch direkt unter Zusatz von Impfkristallen einer Kristallisation unterworfen werden, so daß vorwiegend langkettige Inuline ausfallen und durch Zentrifugation abgetrennt werden können. Die erhaltenen Produkte sind jedoch relativ stark mit den Begleitstoffen, insbesondere den kurzkettigen Inulinen verunreinigt. Auch bei diesem Verfahren bleiben große Anteile der höheren Inuline in der Mutterlauge und gehen so verloren.

Aus BE 92/010921 (unveröffentlicht) ist bekannt, durch eine chromatographische Trennung des Inulins, niedermolekulare Anteile bis etwa DP 5 abzutrennen. Hierbei ist aufgrund der geringen Trennwirkung das erhaltene Inulin mit größeren Mengen an kurzkettigen Molekülen verunreinigt und weist somit eine niedrige mittlere Kettenlänge auf.

Die Anmeldung WO 91/18000 beschreibt ein Verfahren zur Gewinnung von Oligosacchariden aus Biomasse durch Ultrafiltration. Die Anwendung dieses Verfahrens für die Trennung von Inulin hat jedoch einige gravierende Nachteile. Die Mono- und Disaccharide sowie kurzkettige Oligosaccharide werden im Permeat in extrem dünnen Lösungen erhalten und können nur unter hohem Energieaufwand zurückgewonnen werden. Die Membranen sind teuer und haben zudem nur kurze Standzeiten. Sie neigen außerdem stark zu "Fouling".

Es stellte sich daher die Aufgabe, ein neues Verfahren zur Herstellung von langkettigen Inulinen zu finden, welches die Nachteile der bekannten Verfahren vermeidet.

Die Aufgabe wird durch die Merkmale des Hauptanspruchs gelöst und durch die der Unteransprüche gefördert. Überraschenderweise führt die Behandlung einer Rohinulin-Suspension mit geeigneten Hydrolasen unter bestimmten Bedingungen zu einem Produkt, das neben langkettigem Inulin praktisch nur noch Glucose und Fructose enthält, d. h. im wesentlichen frei von kurzkettigen Oligosacchariden ist. Dieses Produkt läßt sich großtechnisch chromatographisch, durch Ultrafiltration oder Kristallisation in eine nur langkettiges Inulin enthaltende Fraktion, eine Mischfraktion aus Glucose und Fructose sowie eine Fructose-Fraktion mit hoher Reinheit, auftrennen. Dadurch wird nicht nur das gewünschte langkettige Inulin sondern auch Fructose als wertvolles Nebenprodukt in hoher Reinheit gewonnen.

Das Prinzip des erfindungsgemäßen Verfahrens, in einer Suspension die überwiegend gelösten mittleren und kurzen Ketten enzymatisch abzubauen und dabei die schwerlöslichen Bestandteile nicht zu spalten, ist nicht auf Inulin beschränkt, sondern läßt sich auch auf andere Polysaccharide, bei denen längerkettige Produkte von den kürzerkettigen getrennt werden sollen, beliebig anwenden.

Bei der Extraktion von inulinhaltigem Pflanzenmaterial wie Zichorienwurzeln bzw. Dahlien- und Topinamburknollen werden Extrakte erhalten, die immer die gesamte Spannbreite von monomeren bis polymeren Kohlenhydraten enthalten. Die niedermolekularen Kohlenhydrate sind weitgehend bereits im Pflanzenmaterial enthalten, können jedoch auch teilweise während der Extraktion sowie bei der Entsalzung des Extraktes zusätzlich entstehen. Die Löslichkeit dieser Kohlenhydrate ist einerseits abhängig von der Temperatur und nimmt andererseits mit zunehmender Kettenlänge ab. Erfindungsgemäß wird die Rohinulinlösung soweit eingedickt, daß eine Suspension entsteht, welche sich noch verarbeiten, d. h. pumpen oder rühren läßt. So lassen sich z. B. bei 50 °C Suspensionen mit 40 Gew.-% Rohinulingehalt problemlos verarbeiten. Durch Erhöhung der Temperatur auf 60 °C läßt sich die Rohinulinkonzentration auf 50 % steigern. Obwohl das zu erhöhter Konsistenz führt, läßt sich die Suspension noch gut verarbeiten.

In Abhängigkeit von der Temperaturstabilität sind Rohinulingehalte von 20 - 70 Gew.-%, vorzugsweise 40 - 50 Gew.-%, sinnvoll. Solche Rohinulin-Suspensionen enthalten bei entsprechender Temperatur und Konzentration alle mono-, di- und oligomeren Kohlenhydrate in gelöster und das langkettige Inulin in suspendierter Form.

Nach einer bevorzugten, jedoch nicht einschränkenden Ausführungsform des erfindungsgemäßen Verfahrens wird eine Rohinulinsuspension dadurch hergestellt, daß man zunächst eine klare, 20 - 70 Gew.-% Rohinulin enthaltende Lösung bei 80 -100 °C herstellt, diese langsam auf 30 - 70 °C, vorzugsweise 40 - 60 °C, abkühlt und die dabei entstandene Suspension für 2-48 Stunden bei dieser letzten Temperatur hält. Die Kühlrate beträgt dabei z. B. 0,1 - 2,5 Kh⁻¹. Weitere Verfahren zur Herstellung solcher Suspensionen sind z.B. Mischen unter hoher Scherung, Mikrowellen- bzw. Ultraschalleinsatz.

Entsprechend einer weiteren bevorzugten Ausführungsform des erfindungsgemässen Verfahrens, kann die klare Rohinulinlösung einer gezielten Kühlungskristallisation mit definierter Kühlrate und unter Verwendung von Impfkristallen unterworfen werden.

Es ist jedoch auch möglich, die Suspension einfach durch Einrühren des Rohinulins in Wasser und mehrstündigem Rühren bei der gewählten Temperatur herzustellen.

Für das erfindugnsgemäße Verfahren ist es nicht erforderlich, daß das Rohinulin in trockener Form vorliegt; der bei der Herstellung des Rohinulins als Zwischenprodukt anfallende entmineralisierte Extrakt ist ebenfalls als Rohstoff geeignet.

Falls erforderlich, wird der pH-Wert der Rohinulinsuspension entsprechend dem pH-Optimum des zu verwendenden Enzyms eingestellt. Für Inulinase (z. B. SP 230 von NOVO mit einer Aktivität von 3000 Inulinase-Einheiten (INU)/g) beträgt dieser Wert 4,5 - 5,0.

Als Enzyme kommen im Falle des Inulins Hydrolasen wie Inulinasen, Exo-Inulinasen oder β-Invertasen in Frage. Gegebenenfalls können auch verschiedene Hydrolasen gemeinsam zum Einsatz kommen.

Die Reaktionstemperatur richtet sich einerseits nach der Temperaturstabilität der verwendeten Enzyme und andererseits nach dem Trockensubstanzgehalt der Rohinulin-Suspension. In der Regel wird die Temperatur je nach Trockensubstanzgehalt zwischen 30 - 70 °C konstant gehalten.

Es kann jedoch auch vorteilhaft sein, die Temperatur während der Reaktion auch zu variieren, z. B. bei abnehmendem Gehalt an mittellangen Ketten zu senken, oder zur Verringerung der Viskosität der Lösung zu erhöhen.

Die Enzymdosierung pro kg Rohinulin richtet sich nach dem Gehalt an kurzkettigen Oligosacchariden, Reaktionstemperatur und der angestrebten Reaktionszeit. Im Falle der Inulinase werden bei 50 - 60 °C je nach der zur Verfügung stehenden Reaktionszeit 500 - 4000 Inulinase-Einheiten pro kg Rohinulin benötigt.

Die Reaktion selbst verläuft unter Rühren und pH-Kontrolle.

Zur Beendigung der enzymatischen Reaktion kann entweder der pH-Wert auf 7,5 - 8,5 angehoben werden und/oder nur die Temperatur auf 90 - 95 °C erhöht werden. Im Hinblick auf die nachfolgende chromatographische Trennung hat es sich als vorteilhaft erwiesen, die Temperatur zu erhöhen und diese dann für 20 - 30 Minuten beizubehalten.

Die so erhaltene klare Lösung kann sofort nach einer an sich bekannten Methode chromatographisch getrennt werden. Wird die Trennung an Ca²⁺- beladenen stark sauren Kationenaustauscherdurchgeführt, ist die Elutionsfolge: langkettiges Inulin, Glucose und Fructose.

Anstelle der Chromatographie kann die Abtrennung des langkettigen Inulins auch mit einer Dekanter-Zentrifuge oder nach einem anderen bekannten Trennverfahren erfolgen. In diesem Fall wird die enzymatische Reaktion nicht durch Temperaturerhöhung sondern nur durch pH-Wert-Anhebung gestoppt. Nach Beendigung der enzymatischen Rektion durch Anhebung des pH-Werts kann das in Suspension befindliche langkettige Inulin auch durch Filtration in geeigneten Apparaturen, wie z. B. Drucknutschen, von den gelösten niedermolekularen Anteilen abgetrennt werden.

Da die nach der enzymatischen Reaktion erhaltene klare Lösung neben langkettigem Inulin im wesentlichen nur Fructose, Glucose und Saccharose enthält, kann eine Abtrennung des langkettigen Inulins auch durch Ultrafiltration durchgeführt werden. Da nur Mono- und Disaccharide von Molekülen mit hohem Polymerisationsgrad (DP > 10) abzutrennen sind, genügt der Einsatz relativ einfacher und dadurch billiger Membranen.

Das erhaltene langkettige Inulin kann durch ein an sich bekanntes Trocknungsverfahren (z. B. Sprühtrocknung) in eine lagerstabile Form gebracht werden.

Das erfindungsgemäße Verfahren erlaubt die Gewinnung von langkettigem Inulin mit einer engen Bandbreite bezüglich der Kettenlänge. Die mittlere Kettenlänge kann in Abhängigkeit von den gewählten Reaktionsbedingungen zwischen 15 und 40 Monomereinheiten, vorzugsweise 20 - 30, liegen. Die Ausbeute an langkettigem Inulin beträgt 20 - 50 % bezogen auf Rohinulin und ist im wesentlichen abhängig vom Gehalt des Inulins mit gewünschter Kettenlänge im ursprünglichen Rohstoff.

Die so hergestellten Inuline werden vorteilhaft als Lebensmittel-Komponente auch zusammen mit Süßungsmitteln, bulking agents, Verdickungsmitteln, Stabilisatoren etc., sowie als Fett-Ersatz in Lebensmitteln, Kohlenhydrat-Ersatz, Diät-Faserstoff und außerdem als Träger für Pharmawirkstoffe und als Indikator für Nieren-Clearence eingesetzt.

### Beispiel 1: Bestimmung der mittleren Kettenlänge

Inulin-Moleküle bestehen aus einer Kette aus Fructose-Molekülen, die endständig jeweils ein Glucose-Molekül trägt. Vollständige enzymatische Hydrolyse des Inulins führt zu Bildung von Fructose und Glucose, wobei das Fructose/Glucose-Verhältnis die mittlere Kettenlänge des Inulins darstellt.

1 g der zu untersuchenden Inulinprobe wird in 100 cm³ 0,1 M Na-Acetat-Puffer, pH 5,0 durch Erhitzen auf 96 °C für 20 Minuten gelöst. 10 cm³ dieser Lösung werden bei 56 °C für 5 Stunden mit 6 Einheiten Inulinase inkubiert.

Die Bestimmung der entstandenen Glucose und Fructose kann mit HPLC bzw. enzymatisch durchgeführt werden.

### Beispiel 2: Herstellung von Rohinulin

100 kg fein zerkleinerte Zichorienwurzeln mit 14,5 kg Inulingehalt werden mit 125 kg Wasser bei 75 °C und pH 5,5 in Gegenstrom extrahiert. Der Extrakt wird zunächst durch Zusatz von 1,24 kg CaO auf pH 10,0 - 12,0 und dann durch Einleiten von CO₂ auf pH-Wert 8,5 eingestellt. Der entstandene Niederschlag aus CaCO₃ und den ausgefällten Verunreinigungen wird durch Filtration abgetrennt.

Die klare Lösung wird durch Ionenaustausch an schwachsauren Kationen- und schwachbasischen Anionenaustauschern entmineralisiert. Daran anschließend wird die Lösung zunächst auf 35 - 40 % Trockensubstanzgehalt eingedampft (im nachfolgenden als Rohinulin flüssig bezeichnet) und dann in einer geeigneten Trocknungseinrichtung (z.B. ein Sprühtrockner) in trockene Form überführt.
- Ausbeute:: 10 kg Rohinulin trocken
- Mittlere Kettenlänge:: 7 - 9

Diese Arbeitsweise ist nicht auf die Verarbeitung von Zichorien beschränkt. Nach der gleichen Methode können auch andere Inulin enthaltende Pflanzen bzw. Pflanzenteile verarbeitet werden.

In den nachfolgenden Beispielen kann sowohl mit getrocknetem Rohinulin als auch mit dem entmineralisierten, auf 35 - 40 % TS-Gehalt eingedampften Extrakt gearbeitet werden, da beide Produkte sich als Rohstoff für die Gewinnung von langkettigem Inulin gleich gut eignen.

### Beispiel 3: Stand der Technik; Inulinfraktionierung durch Ausfällung mit Lösungsmittel

2,25 kg Rohinulin flüssig mit 40 % TS-Gehalt bzw. 0,9 kg Rohinulin trocken werden mit 4,5 l bzw. 6,0 l Wasser aufgelöst und unter langsamem Rühren mit 4 l Isopropanol versetzt. Der Niederschlag wird über Nacht absetzen gelassen. Der Überstand wird abdekantiert und dann der Niederschlag durch Zentrifugation abgetrennt. Aus dem Überstand wird Isopropanol durch Destillation zurückgewonnen, mit Wasser auf 40 %-iges Isopropanol eingestellt und damit der Niederschlag wiederholt gewaschen.

Der gewaschene Niederschlag wird über Nacht unter Vakuum bei 40 °C getrocknet.

- Ausbeute:: 28 %, bezogen auf eingesetztes Rohinulin
- Mittlere Kettenlänge:: 17

### Beispiel 4: Stand der Technik; Inulinfraktionierung durch Ultrafiltration

20 g Rohinulin trocken (oder eine entsprechende Menge Rohinulin flüssig) wurden zunächst mit 2 l Wasser verdünnt und in eine Ultrafiltrationsanlage bestückt mit einem Membran mit 500 Dalton Trenngrenze bei Raumtemperatur getrennt. Das Retentat wurde zweimal mit jeweils 600 cm³ Wasser verdünnt und ultrafiltriert. Danach wurde das Retentat zur Trockne eingedampft.

- Ausbeute:: 30 %, bezogen auf eingesetztes Rohinulin
- Mittlere Kettenlänge:: 18

### Beispiel 5: Stand der Technik; Inulinfraktionierung durch Kristallisation

20 kg Rohinulin trocken wurden in 40 l Wasser suspendiert und durch Erhitzen auf 80 °C in Lösung gebracht (entsprechende Menge Rohinulin flüssig kann direkt eingesetzt werden) und dann langsam auf 50 °C gekühlt. Dabei fiel ein Teil des Inulins in fester Form aus. Eine Korngrößenmessung ergab die mittlere Korngröße als 20 µm. Da diese Partikelgröße für eine Trennung an eine Siebkorbzentrifuge zu klein ist, wurde die Trennung an einer Dekanterzentrifuge durchgeführt. Der Rückstand wurde dabei mit Wasser bei 50 °C gewaschen und danach getrocknet.

- Ausbeute:: 22 %, bezogen auf eingesetztes Rohinulin
- Mittlere Kettenlänge:: 12

### Beispiel 6: Stand der Technik; Inulinfraktionierung durch Chromatographie

Eine Chromatographiesäule mit 10 m Länge und 0,25 m Durchmesser, gefüllt mit einem Ca²⁺-beladenen schwachvernetzten starksauren Kationenaustauscherharz (z.B. Duolite^{R} C 204), wurde auf 60 °C temperiert, mit 15 kg Rohinulin als 40 Gew.-%-ige wässrige Lösung beaufschlagt und mit auf pH 9,0 alkalisiertem (mit Ca(OH)₂) entsalztem Wasser eluiert. Das Effluat wurde so geschnitten, daß eine Fraktion mit DP 5 und höheren Homologen und eine zweite Fraktion mit DP < 5 gesammelt wurde.

Die Fraktion mit DP > 5 enthielt das langkettige Inulin. Diese wurde zur Trockne eingedampft. Die Ausbeute und mittlere Kettenlänge dieses Produkts sind von der Lage des gewählten Schnittpunkts abhängig.

- Ausbeute:: 25 - 35 %, bezogen auf eingesetztes Rohinulin
- Mittlere Kettenlänge:: 12 - 16

### Beispiel 7:

16 kg Rohinulin trocken wurden in einen temperierbaren Rührkessel in 24 l Wasser suspendiert (eine entsprechende Menge Rohinulin flüssig kann direkt eingesetzt werden) und unter Rühren auf 50 °C temperiert. Nach einer Verweilzeit von 8 h wurde der pH-Wert auf 4,8 - 5,0 eingestellt und 18 ml Inulinase (NOVO, SP 230) hinzugefügt. Während der Inkubationszeit von 24 h wurde die Temperatur auf 50 °C und der pH-Wert auf 4,8 - 5,0 konstant gehalten.

Durch Aufheizen der Suspension auf 95 °C wurde die enzymatische Reaktion beendet und gleichzeitig die suspendierten Partikel in Lösung gebracht. Die klare Lösung wurde mit einer Chromatographieanlage, wie unter Beispiel 6 beschrieben, chromatographiert. Die langkettiges Inulin enthaltende Fraktion wurde zur Trockne eingedampft.

| | | |
|---|---|---|
| Ausbeute: | | |
| langkettiges Inulin: | 4,8 kg | = 30 %, bezogen auf Rohinulin |
| Fructose: | 8,8 kg | = 55 %, bezogen auf Rohinulin |
| Mittlere Kettenlänge: | 16 | |

### Beispiel 8:

16 kg Rohinulin trocken wurden in einen temperierbaren Rührkessel in 16 l Wasser suspendiert (eine entsprechende Menge Rohinulin flüssig kann nach Eindampfung auf 50 % TS-Gehalt eingesetzt werden) und unter Rühren zunächst auf 80 - 90 °C aufgeheizt und dadurch in Lösung gebracht. Anschließend wurde die Temperatur schrittweise auf 60 °C abgesenkt und die entstandene Suspension für 16 Stunden bei dieser Temperatur belassen. Nach pH-Einstellung auf 4,8 - 5,0 wurde die Suspension mit 18 ml Inulinase (NOVO, SP 230) versetzt und 24 h bei 60 °C und pH 4,8 - 5,0 unter Rühren inkubiert.

Durch Aufheizen der Suspension auf 95 °C wurde die enzymatische Reaktion beendet und gleichzeitig die suspendierten Partikel in Lösung gebracht.

Die klare Lösung wurde durch Chromatographie entsprechend Beispiel 6 getrennt. Die Fraktion mit langkettigem Inulin wurde zur Trockne eingedampft, während die Fructosefraktion nur bis 70 % TS-Gehalt konzentriert zu werden brauchte. Die erreichte mittlere Kettenlänge des Inulinprodukts sowie Ausbeute sind abhängig von der Temperatur sowie Dauer der enzymatischen Reaktion.

| | |
|---|---|
| Ausbeute: | |
| langkettiges Inulin: | 30 - 45 % des eingesetzten Rohinulins |
| Fructose: | 45 - 60 % des eingesetzten Rohinulins |
| Mittlere Kettenlänge: | 25 - 40 |

### Beispiel 9:

Es wurde ein Ansatz entsprechend Beispiel 8 enzymatisch behandelt. Die enzymatische Reaktion wurde durch Alkalisierung auf pH 8,5 beendet.

Die Suspension wurde bei 60 °C in einer Ultrafiltrationsanlage über eine Membran mit 500 Dalton Trenngrenze getrennt. Das Permeat wurde eingedickt und zur Fructosegewinnung eingesetzt. Das langkettiges Inulin enthaltende Retentat wurde zur Trockne eingedampft.

| | |
|---|---|
| Ausbeute: | |
| langkettiges Inulin: | 30 - 45 % des eingesetzten Rohinulins |
| Fructose: | 45 - 60 % des eingesetzten Rohinulins |
| Mittlere Kettenlänge: | 25 - 40. |

## Patentansprüche

1. Verfahren zur Herstellung von langkettigem Inulin bei gleichzeitiger Gewinnung von Glucose und Fructose **dadurch gekennzeichnet,** daß eine wässrige Rohinulinsuspension mit einer Rohinulinkonzentration von 20 - 70 Gew.-% enzymatisch mit Hydrolasen bei Temperaturen von 30 - 70 °C behandelt wird, wobei die kurzkettigen Anteile zu Mono- und Disacchariden abgebaut werden, und wobei die langkettigen Inuline im wesentlichen frei von Molekülen mit einem Polymerisationsgrad < 10-12 sind und von den Mono- und Disacchariden getrennt und in trockene Form überführt werden.

2. Verfahren nach Anspruch 1, **dadurch gekennzeichnet,** daß die Rohinulinkonzentration 40 - 50 Gew.-% beträgt.

3. Verfahren nach Anspruch 1 oder 2, **dadurch gekennzeichnet,** daß die Rohinulin-Suspension vor der enzymatischen Behandlung auf 80 - 95 °C aufgeheizt, dann auf 30 - 70 °C temperiert und während der enzymatischen Behandlung konstant auf einer Temperatur zwischen 30 und 70 °C gehalten wird.

4. Verfahren nach einem der Ansprüche 1 bis 3, **dadurch gekennzeichnet,** daß die enzymatische Behandlung der Rohinulin-Suspension mit Hydrolasen wie Inulinasen, Exo-Inulinasen oder β-Invertasen oder Mischungen davon bei für das Enzym geeigneten Temperaturen und pH-Werten durchgeführt wird.

5. Verfahren nach einem der Ansprüche 1 bis 4, **dadurch gekennzeichnet,** daß die enzymatische Behandlung in Abhängigkeit von der Enzymdosierung nach einer Reaktionszeit von 2 - 48 h durch Erhöhung des pH-Wertes auf 8,0 bis 9,0 oder Aufheizen der Mischung auf 90 - 100 °C beendet wird.

6. Verfahren nach einem der Ansprüche 1 bis 5, **dadurch gekennzeichnet,** daß das langkettige Inulin aus der Reaktionslösung durch Kühlungskristallisation und Zentrifugation isoliert und nach an sich bekannten Methoden in trockene Form überführt wird.

7. Verfahren nach einem der Ansprüche 1 bis 6, **dadurch gekennzeichnet,** daß die erhaltene Inulinsuspension durch Erwärmen in eine klare Lösung überführt und über Ca²⁺-beladene, stark saure Kationenaustauscherharze chromatographisch in
eine reine Fructose-Fraktion,
eine Glucose/Fructose-Mischfraktion und
eine langkettige Inulin-Fraktion
aufgetrennt wird, und daß die Fraktion mit langkettigem Inulin nach an sich bekannten Methoden in trockene Form überführt wird.

8. Verfahren nach einem der Ansprüche 1 bis 6, **dadurch gekennzeichnet,** daß die erhaltene Suspension durch Verdünnen gelöst und durch Ultrafiltration in eine Mono- und Disaccharid-Fraktion und eine langkettige Inulin-Fraktion aufgetrennt wird, und daß die Fraktion mit langkettigem Inulin nach an sich bekannten Methoden in trockene Form überführt wird.

9. Langkettiges Inulin mit einer mittleren Kettenlänge von mehr als 20, welches im wesentlichen frei ist von Sacchariden mit einer Kettenlänge unter 10.

10. Verwendung von Inulinen gemäß Anspruch 9 in Lebensmitteln, Diätmitteln und als Träger von Arzneimitteln.

## Claims

1. Method for the preparation of long-chain inulin while simultaneously obtaining glucose and fructose, characterized in that an aqueous crude inulin suspension with a crude inulin concentration of 20 - 70% by weight is treated enzymatically with hydrolases at temperatures of 30 - 70°C, with the short-chain components being broken down into monosaccharides and disaccharides, and with the long-chain inulins being substantially free of molecules with a degree of polymerization < 10 - 12 and being separated from the monosaccharides and disaccharides and being converted into a dry form.

2. Method according to claim 1, characterized in that the crude inulin concentration amounts to 40 - 50% by weight.

3. Method according to claim 1 or 2,
characterized in that the crude inulin suspension is heated before the enzymatic treatment to 80 - 95°C, is then tempered to 30 - 70°C and during the enzymatic treatment is kept constant at a temperature between 30 and 70°C.

4. Method according to one of claims 1 to 3,
characterized in that the enzymatic treatment of the crude inulin suspension is carried out with hydrolases such as inulinases, exo-inulinases or β-invertases or mixtures thereof at temperatures and pH-values suitable for the enzyme.

5. Method according to one of claims 1 to 4,
characterized in that the enzymatic treatment in dependence upon the enzyme dosage is ended after a reaction time of 2 - 48 h by increasing the pH-value to 8.0 to 9.0 or heating the mixture to 90 - 100°C.

6. Method according to one of claims 1 to 5,
characterized in that the long-chain inulin is isolated from the reaction solution by cooling crystallization and centrifugation and is converted into a dry form in accordance with methods known in themselves.

7. Method according to one of claims 1 to 6,
characterized in that the inulin suspension obtained is converted by heating into a clear solution and is chromatographically separated by way of Ca²⁺-loaded, greatly acidic cationic exchange resins into
a pure fructose fraction,
a glucose/fructose mixed fraction and
a long-chain inulin fraction,
and in that the fraction with long-chain inulin is converted into a dry form in accordance with methods known in themselves.

8. Method according to one of claims 1 to 6,
characterized in that the suspension obtained is released by dilution and is separated by way of ultrafiltration into a monosaccharide and disaccharide fraction and a long-chain inulin fraction, and in that the fraction with long-chain inulin is converted into a dry form according to methods known in themselves.

9. Long-chain inulin with an average chain length of more than 20, which is substantially free of saccharides with a chain length below 10.

10. Use of inulins according to claim 9 in food, dietary agents and as carriers of medicines.

## Revendications

1. Procédé de fabrication d'inuline à longue chaîne avec obtention simultanée de glucose et de fructose, caractérisé en ce qu'il est traité, de manière enzymatique, une suspension d'inuline brute aqueuse avec une concentration d'inuline brute de 20 à 70 % en poids, avec des hydrolases à des températures de 30 à 70 °C, les fractions à courte chaîne étant désintégrées en monosaccharides et disaccharides et les inulines à longue chaîne sont sensiblement libres de molécules avec un degré de polymérisation < 10 à 12 et sont séparées des monosaccharides et disaccharides et sont transformées dans une forme sèche.

2. Procédé selon la revendication 1, caractérisé en ce que la concentration d'inuline brute est de 40 à 50 % en poids.

3. Procédé selon la revendication 1 ou 2, caractérisé en ce que la suspension d'inuline brute est chauffée avant le traitement enzymatique à une température comprise entre 80 et 95 °C, puis est tempérée de 30 à 70 °C et pendant le traitement enzymatique est maintenue constante à une température comprise entre 30 et 70 °C.

4. Procédé selon l'une des revendications 1 à 3, caractérisé en ce que le traitement enzymatique de la suspension d'inuline brute est réalisé avec des hydrolases telles que des inulinases, des exo-inulinases ou des invertases β ou des mélanges de celles-ci, à des températures et des valeurs de pH convenant à l'enzyme.

5. Procédé selon l'une des revendications 1 à 4, caractérisé en ce que le traitement enzymatique est terminé en fonction du dosage d'enzymes après un temps de réaction de 2 à 48 heures, par augmentation du pH de 8 à 9 ou chauffage du mélange à une température comprise entre 90 et 100 °C.

6. Procédé selon l'une des revendications 1 à 5, caractérisé en ce que l'inuline à longue chaîne est isolée de la solution de réaction, par cristallisation par refroidissement et centrifugation et est transformée sous une forme sèche, selon des méthodes connues.

7. Procédé selon l'une des revendications 1 à 6, caractérisé en ce que la suspension d'inuline obtenue est transformée par chauffage en une solution claire et est séparée, de manière chromatographique, par des résines d'échangeurs de cations, fortement acides, chargées de Ca²⁺, en
une pure fraction de fructose,
une fraction mixte glucose/fructose et
une fraction d'inuline à longue chaîne
et en ce que la fraction avec inuline à longue chaîne est transformée dans une forme sèche, suivant des méthodes connues.

8. Procédé selon l'une des revendications 1 à 6, caractérisé en ce que la suspension obtenue est dissoute par dilution et séparée par ultrafiltration en une fraction de monosaccharide et disaccharide et une fraction d'inuline à longue chaîne, et en ce que la fraction avec inuline à longue chaîne est transformée en une forme sèche, suivant des méthodes connues.

9. Inuline à longue chaîne avec une longueur de chaîne moyenne de plus de 20, qui est essentiellement libre de saccharides avec une longueur de chaîne inférieure à 10.

10. Utilisation d'inulines selon la revendication 9 dans des produits alimentaires, des produits diététiques et comme supports de médicaments.
